Europäisches Patentamt

European Patent Office

Office européen des brevets

(19)

(11) Veröffentlichungsnummer: **0 140 123**

**A1**

(12) # EUROPÄISCHE PATENTANMELDUNG

(21) Anmeldenummer: **84111103.2**

(22) Anmeldetag: **18.09.84**

(51) Int. Cl.⁴: **C 07 C 25/08**
**C 07 C 17/24**

(30) Priorität: **24.09.83 DE 3334673**

(43) Veröffentlichungstag der Anmeldung:
**08.05.85 Patentblatt 85/19**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Litterer, Heinz, Dr.**
**Hardtstrasse 77**
**D-6208 Bad Schwalbach(DE)**

(54) **Verfahren zur Herstellung von m-Dichlorbenzol.**

(57) Die Erfindung betrifft ein Verfahren zur Herstellung von m-Dichlorbenzol durch Isomerisierung von o- oder p-Dichlorbenzol oder deren Gemisch an einem Zeolithkatalysator, vorzugsweise in der Gasphase. Die Zeolithe sind vorzugsweise ionenausgetauscht.

EP 0 140 123 A1

- 1 -

HOECHST AKTIENGESELLSCHAFT          Dr.MA/AK      HOE 83/F 202

Verfahren zur Herstellung von m-Dichlorbenzol
_____

Die vorliegende Erfindung betrifft ein Verfahren zur Herstellung von m-Dichlorbenzol durch Isomerisierung von o-
oder p-Dichlorbenzol oder deren Gemisch.

Bekanntlich werden Dichlorbenzole durch Chlorierung von
Benzol mit Chlor in Gegenwart spezieller Lewissäurekatalysatoren gewonnen.
Gemeinsam ist den technisch gangbaren Verfahren der
geringe Anteil an m-Dichlorbenzol, der dabei entsteht.
So erhält man unter milden Reaktionsbedingungen bei 30 -
50°C in Gegenwart von Eisenchlorid und Chlorschwefelverbindungen ein Produkt, das aus 74,8 % p-, 25 % o- und
0,2 % m-Dichlorbenzol besteht.

Bei stärkerer Aktivierung mit Aluminium-, Antimon- oder Molybdänchlorid   und Reaktionstemperaturen von 60 - 100°C
stellt sich hingegen ein Isomerenverhältnis   von ca. 56 % p-,
2 % m- und 42 % o-Dichlorbenzol ein (Ullmann, Band 9,
Seite 507, 1975).
Dieser Tatsache überlagert sich jedoch ein unterschiedlicher
Marktbedarf an Dichlorbenzolen, speziell an m-Dichlorbenzol.

Die bisher bekannten Wege zur Herstellung von m-Dichlorbenzol
aus o- oder p-Dichlorbenzol sind technisch oder wirtschaftlich sehr aufwendig oder im größeren Maßstab nicht gangbar.

So wird die Isomerisierung eines Dichlorbenzolgemisches in Gegenwart von 10 % $AlCl_3$ und 1 % $H_2O$ bei 150 - 200°C beschrieben. (DE-PS 873 695, DE-PS 926 185). Dabei treten jedoch die üblichen Probleme mit einer hochkorrosiven und verfahrenstechnisch schwierig zu handhabenden Lewis-Säure auf. Außerdem ist das Verfahren wenig selektiv.

Eine weitere, jedoch aufwendige und zugleich unzureichende Methode zur Gewinnung von m-Dichlorbenzol stellt die Destillation der oben erwähnten, bis zu etwa 2 Gew.% meta-Isomeres enthaltenden Benzol-Chlorierungsprodukte dar.

Gegenstand der vorliegenden Erfindung ist ein Verfahren zur Herstellung von m-Dichlorbenzol durch Isomerisierung von o- oder p-Dichlorbenzol oder deren Gemisch, dadurch gekennzeichnet, daß man einen Zeolithkatalysator verwendet.

Die bei dem erfindungsgemäßen Verfahren eingesetzten Zeolithe sind vor allem natürliche oder synthetische kristalline Alumosilikate.

Grundbausteine dieser kristallinen Zeolithe sind $SiO_4$ und $AlO_4^-$-Tetraeder, die über Sauerstoffatome verbrückt sind und so regelmäßige Strukturen mit Hohlräumen und Porenöffnungen entstehen lassen. Die negativen Ladungen der $AlO_4^-$-Tetraeder werden durch Kationen wie $Na^+$, $K^+$, $Ca^{2+}$, $Mg^{2+}$ oder organische Kationen wie z.B. $NR_4^+$ kompensiert.

Die synthesebedingt vorhandenen Kationen können nach den üblichen Methoden gegen andere Kationen ausgetauscht werden, so daß Katalysatoren oder Adsorbentien mit sehr unterschiedlichen Eigenschaften erhalten werden (D.W. Breck: Zeolite Molecular Sieves, 1974). Die Modifizierung kann auch, wie im Falle von Zeolithtypen, die organischen Kationen wie $NR_4^+$ oder $PR_4^+$ enthalten, durch eine thermische Behandlung erreicht werden.

Für das erfindungsgemäße Verfahren geeignete Zeolithe - dem Fachmann als Adsorbentien oder als Katalysatoren für Kohlenwasserstoffumwandlungen bekannt - sind u.a. Faujasite, Mordenite, Ferrierite sowie Zeolithe mit Pentasil-Struktur. Beispielsweise eignen sich die synthetischen Zeolithe X (US-PS 2 882 244), Y (US-PS 3 130 007), ZSM-5 (US-PS 3 702 886), ZSM-11 (US-PS 3 709 979), ZSM-8 (GB-PS 1 334 243), ZSM-12 (US-PS 3 832 449), ZSM-20 (US-PS 3 972 983), ZSM-21 (US-PS 4 046 859), ZSM-23 (US-PS 4 076 842), ZSM-35 (US-PS 4 016 245), ZSM-38 (US-PS 4 046 859), ZSM-48 (EPA 0 034 918).

Für den Begriff Pentasile gilt dabei die Definition von Kokotailo und Meier ("Pentasil family of high silicon crystalline materials" in Special Publication No. 33 of the Chemical Society, London, 1980).

Besonders geeignet für das vorliegende Verfahren sind die synthetischen Zeolithe des Pentasiltyps, wie ZSM-5, ZSM-8, ZSM-11 und ZSM-48, sowie die Zeolithe ZSM-12, ZSM-20 und Mordenit.

Das Si/Al-Verhältnis dieser Zeolithe liegt im allgemeinen zwischen 4 und 2000, vorzugsweise zwischen 10 und 400. Der Aluminiumgehalt kann zum Zwecke einer weitergehenden Modifizierung durch Behandlung mit Mineralsäuren, organischen Säuren oder chelatisierenden Substanzen vermindert werden, was eine Steigerung der Standzeit dieser Katalysatoren bewirkt.

Für das erfindungsgemäße Verfahren sind auch Zeolithe geeignet, bei denen Al, beziehungsweise Si, durch andere Gitteratome wie Bor, Eisen, Gallium, Germanium, Titan oder Zirkon ersetzt sind.

Die genannten Zeolithe werden für den technischen Einsatz mit Hilfe von Bindern in Strangform gebracht.
Als Bindermaterialien sind vor allem die Oxide, Hydroxide
oder Hydroxychloride des Aluminiums und die Oxide des
Siliciums, die Oxide des Titans und Zirkons sowie Tonmaterialien geeignet.

Vorzugsweise werden die Zeolithe für das erfindungsgemäße
Verfahren  in der Weise modifiziert, daß man sie durch Ionenaustausch mit ein-, zwei- oder dreiwertigen Kationen, vorzugsweise mit $NH_4^+$, $H^+$, $Be^{2+}$, $Mg^{2+}$, $Ca^{2+}$, $La^{3+}$ oder Selten-
erd-Kationen oder mit Kombinationen dieser Elemente in
die katalytisch aktive Form überführt.

Die Zeolith-Katalysatoren werden außerdem vorzugsweise in
der üblichen Art durch Calcinieren aktiviert, was eine
weitere Art der Modifikation darstellt. Gelegentlich ist
es vorteilhaft, Ionenaustausch und Calcinierung mehrmals
zu wiederholen. Die Calcinierung wird vorzugsweise bei
350 bis 700°C durchgeführt. Zur besseren Stabilisierung ist
es manchmal vorteilhaft, die Calcinierung in Gegenwart
von Wasserdampf, Ammoniak oder deren Mischungen bei Temperaturen zwischen 600 und 900°C durchzuführen.

Die Isomerisierung kann sowohl in der Gasphase als auch in
der Flüssigphase durchgeführt werden, wobei die Gasphase
bevorzugt ist. Auch andere Verfahrensweisen, wie beispielsweise eine Rieselphase bei Anwendung eines höheren Druckes
sind möglich.

Wird die erfindungsgemäße Isomerisierung in der Gasphase mit
fest angeordnetem Katalysator durchgeführt, so betragen
die Reaktionstemperaturen im allgemeinen 250 - 600°C, vorzugsweise 300 - 550°C, und die Drücke 1 bis 20 bar, vorzugsweise 2 bis 10 bar.

In der Flüssigphase wird die Isomerisierung bei Temperaturen
von 150 - 350°C, vorzugsweise 200 - 300°C, und bei Drücken
von 1 bis 100 bar, vorzugsweise 5 bis 50 bar durchgeführt.

Die Belastung (WHSV = Weight-Hourly-Space-Velocity $h^{-1}$)
sollte vorzugsweise zwischen 0,1 und 10 $h^{-1}$ liegen.

Zusätzlich zu den zu isomerisierenden Dichlorbenzolen
können zur Herabsetzung des Partialdrucks ggf. inerte Löse-
oder Verdünnungsmittel, wie $N_2$ oder $H_2$ über den Katalysator
geleitet werden.

Wird eine Regenerierung des Katalysators erforderlich, so
kann sie durch kontrolliertes Abbrennen mit sauerstoffhaltigen Gasen erfolgen.

Die Isomerisierung nach dem erfindungsgemäßen Verfahren
verläuft sehr selektiv und ergibt in Abhängigkeit von den
Prozessbedingungen, wie z.B. Temperatur, Verweilzeit sowie
in Abhängigkeit vom Zeolithtyp hohe Anteile an meta-Dichlorbenzol.

Vorzugsweise wird bei dem erfindungsgemäßen Verfahren das
bei der zu Beginn erwähnten Chlorierung von Benzol mit
Lewis-Säuren entstehende, geringe meta-Anteile enthaltende
Rohprodukt über einen Zeolithkatalysator geleitet und isomerisiert. 
Eine andere Ausführungsform des erfindungsgemäßen Verfahrens
besteht darin, daß nur o-Dichlorbenzol über den Zeolithkatalysator geleitet und isomerisiert wird. Das hierbei
anfallende meta-reiche Isomerengemisch wird dann in bekannter Weise (Ullmann, Band 9, Seite 507, 1975) zur Gewinnung von m-Dichlorbenzol aufgearbeitet und das nichtumgesetzte o-Dichlorbenzol sowie das als Nebenprodukt entstandene p-Dichlorbenzol in den Isomerisierungsreaktor
zurückgeführt. Gegebenenfalls wird aus dem genannten orthopara-Gemisch das para-Isomere abgetrennt und nur das ortho-
Dichlorbenzol erneut der Isomerisierung zugeführt.

Das erfindungsgemäße Verfahren kann sowohl kontinuierlich
als auch diskontinuierlich betrieben werden.

Die nachfolgenden Beispiele sollen die Erfindung näher erläutern.

Beispiel 1:

Ein gemäß US-PS 3 702 886 synthetisierter Zeolith ZSM-5 wird nach einer 14-stündigen thermischen Vorbehandlung im Verhältnis 2 : 1 mit Aluminiumoxid vermischt, mit einer angesäuerten wäßrigen Lösung angeteigt und zu 1.4 mm starken Extrudaten verarbeitet. Die erhaltenen Katalysatorstränge werden anschließend eine Stunde bei 120°C getrocknet und danach 2 Stunden bei 600°C geglüht. Nach dieser Behandlung werden die Stränge mehrmals mit einer 10 %igen Ammonsulfatlösung in Kontakt gebracht und die synthesebedingt vorhandenen Natriumionen gegen $NH_4^+$ erschöpfend ausgetauscht. Durch eine nochmalige thermische Behandlung dieser Stränge bei 600°C erhält man die Wasserstofform des Zeolithen ZSM-5.

Zur Isomerisierung eines Benzol- Rohchlorierungsproduktes (74 % p-, 25 % o-, 1 % m-Dichlorbenzol) in ein meta-reiches Isomerengemisch wird ein Festbettreaktor von 20 mm Innendurchmesser und 1000 mm Länge mit 100 ml des oben beschriebenen Katalysators beladen und bei Atmosphärendruck und 400°C mit 150 ml pro Stunde des Rohchlorierungsprodukts beschickt. Zur Erniedrigung des Einlaßpartialdrucks werden zusätzlich 120 l $N_2$/h als Inertgas zudosiert. Die im Reaktoraustrag ermittelte Isomerenverteilung mit 24 % o-Dichlorbenzol, 27 % m-Dichlorbenzol und 48 % p-Dichlorbenzol läßt einen beachtlichen Anstieg des meta-Isomerengehaltes erkennen. Führt man diesen Versuch bei 450°C durch, so ergibt sich folgende Isomerenverteilung: 19 % o-Dichlorbenzol, 52 % m-Dichlorbenzol und 28 % p-Dichlorbenzol.

In beiden Fällen liegen die Selektivitätswerte bezüglich der Bildung von m-Dichlorbenzol bei über 96 %.

Beispiel 2:

Setzt man an Stelle des in Beispiel 1 verwendeten Benzol-Rohchlorierungsproduktes 98 %iges o-Dichlorbenzol ein und behält die sonstigen Versuchsbedingungen bei, so beträgt der o-Dichlorbenzolumsatz 30 %. Die Selektivitätswerte für die Bildung von m-Dichlorbenzol und p-Dichlorbenzol liegen bei 73 bzw. 26 %.

Eine weitere Temperaturerhöhung auf 450°C führt zu einem Umsatzanstieg auf 60 %. Die Selektivitätswerte für m-Dichlorbenzol (67 %) und p-Dichlorbenzol (32 %) weisen eine unverändert hohe Gesamtselektivität des Isomerisierungskatalysators aus.

Beispiel 3:

Dieses Beispiel soll den Einfluß der Katalysatorbelastung und der Reaktionstemperatur auf die Isomerenbildung bei Einsatz von o-Dichlorbenzol zeigen.
Der in Beispiel 1 beschriebene Festbettreaktor wird mit einer LHSV($h^{-1}$), von 4.0 und einem Verhältnis o-Dichlorbenzol: Stickstoff von 1 : 4 (mol/mol) bei 370°C betrieben. Ebenso wird der in Beispiel 1 beschriebene H-ZSM-5 als Katalysator eingesetzt.
Die Analyse des Reaktoraustrages ergibt einen 20 %igen o-Dichlorbenzolumsatz bei einer m-Dichlorbenzol-Selektivität von 82 % und einer p-Dichlorbenzol-Selektivität von 16 %.
Destilliert man m- und p-Dichlorbenzol von überschüssigem o-Dichlorbenzol ab, so können m-Dichlorbenzolfraktionen mit mehr als 85 %igem Gehalt an m-Dichlorbenzol durch einfache Destillation gewonnen werden.

Beispiel 4:

Setzt man an Stelle eines Zeolithen der Serie ZSM-5 oder ZSM-11 die H-Form des Zeolithen ZSM-12 ein und betreibt den Festbettreaktor mit einer Belastung (LHSV) von $0.7 \, h^{-1}$ und bei 370°C unter Zusatz von 100l Stickstoff pro Stunde, so werden an diesem Katalysator 50.7 % des eingesetzten o-Dichlorbenzols umgesetzt. Die Selektivitätswerte ergeben sich zu 73 % für das m-Dichlorisomere und zu 26.8 % für das p-Dichlorisomere.

Eine Temperaturerhöhung auf 410°C führt zu einem Umsatzanstieg auf 77 %. Aus der resultierenden Isomerenverteilung (23.3 % o-Dichlorbenzol, 49.6% m-Dichlorbenzol, 25.5 % p-Dichlorbenzol) und den minimalen Nebenproduktanteilen errechnen sich Selektivitätswerte von 64.6 % für das meta-Isomere und 33.2 % für das para-Isomere.

Beispiel 5:

Verwendet man an Stelle eines Zeolithen ZSM-12 die unter dem Handelsnamen Zeolon 900 H (Norton) käufliche H-Form des Zeolithen Mordenit und fährt diesen Katalysator unter den gleichen Versuchsbedingungen wie in Beispiel 4, so werden 47 % des eingesetzten o-Dichlorbenzols in meta- und para-Isomeres umgewandelt. Die Selektivitätswerte für das meta- und das para-Isomere ergeben sich zu 69 bzw. 29.6 %.

Beispiel 6:

An Hand dieses Beispiels soll der Einfluß des Zeolithtyps auf Umsatz und Selektivitätsverteilung gezeigt werden.

Ein mit 2n HCl behandelter Zeolith vom Ferrierit-Typ (ZSM-35) wird mit $Al_2O_3$ abgebunden und in Form von 1.4 mm starken Strängen in den in Beispiel 1 erwähnten Reaktor eingebracht. Betreibt man den Reaktor mit o-Dichlorbenzol bei einer Belastung (LHSV, $h^{-1}$) von 0.4 und bei einer Reaktionstemperatur von 400°C, so werden 5.8 % des eingesetzten Dichlorbenzols umgesetzt. Es bilden sich hierbei 81.7 % para-Isomeres und 16,9 % meta-Isomeres.

0140123

## Patentansprüche

1. Verfahren zur Herstellung von m -Dichlorbenzol durch Isomerisierung von o - oder p -Dichlorbenzol oder deren Gemisch, dadurch gekennzeichnet, daß man einen Zeolithkatalysator verwendet.

2. Verfahren nach Anspruch 1, dadurch gekennzeichnet, daß man als Zeolithkatalysator einen Faujasit, Mordenit, Ferrierit oder einen Zeolith mit Pentasil-Struktur einsetzt.

3. Verfahren nach Anspruch 1, dadurch gekennzeichnet, daß man Zeolith X, Zeolith Y, Mordenit, ZSM-5, ZSM-11, ZSM-8, ZSM-12, ZSM-20, ZSM-21, ZSM-23, ZSM-35, ZSM-38 oder ZSM-48 verwendet.

4. Verfahren nach Anspruch 1, dadurch gekennzeichnet, daß man Zeolithe mit Pentasil-Struktur oder ZSM-12, ZSM-20 oder Mordenit verwendet.

5. Verfahren nach einem der Ansprüche 1-4, dadurch gekennzeichnet, daß die Zeolithe ionenausgetauscht sind und als Kationen $H^+$, $NH_4^+$, $Be^{2+}$, $Mg^{2+}$, $Ca^{2+}$, $La^{3+}$ oder Seltenerdmetallionen oder Kombinationen dieser Kationen enthalten.

6. Verfahren nach einem der Ansprüche 1-5, dadurch gekennzeichnet, daß man das Verfahren in der Gasphase durchführt.

0140123

Nummer der Anmeldung

**EUROPÄISCHER RECHERCHENBERICHT**

Europäisches
Patentamt

| | EINSCHLÄGIGE DOKUMENTE | | EP 84111103.2 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | JP - A - 58-144 330 (TORAY K.K.)<br><br>  * Beispiele 1,6; Tabelle 1;<br>    Seite 137(3), rechte Spalte,<br>    Zeilen 19-21 * | 1-6 | C 07 C 25/08<br>C 07 C 17/24 |
| P,X | & PATENT ABSTRACTS OF JAPAN, un-<br>examined applications, Sektion C,<br>Band 7, Nr. 259, 18. November<br>1983<br><br>THE PATENT OFFICE JAPANESE<br>Government,<br>Seite 166 C 195<br><br>-- | | |
| A | EP - A1 - 0 062 261 (TORAY INDU-<br>STRIES)<br><br>  * Anspruch 1; Beispiel 1 *<br><br>---- | 1-6 | |
| | | | RECHERCHIERTE SACHGEBIETE (Int. Cl.4) |
| | | | C 07 C 25/00<br>C 07 C 17/00 |

Der vorliegende Recherchenbericht wurde für alle Patentansprüche erstellt.

| Recherchenort | Abschlußdatum der Recherche | Prüfer |
|---|---|---|
| WIEN | 14-12-1984 | KÖRBER |